# EUROPEAN PATENT SPECIFICATION

(11) **EP 4 078 989 B1**
(45) Date of publication and mention of the grant of the patent: **11.11.2026**
(21) Application number: 20829608.7
(22) Date of filing: 18.12.2020
(51) Int. Cl.: H04R 1/10, H04R 1/32, A61F 11/06, G01S 3/808

(54) **HEARING PROTECTION APPARATUS WITH CONTEXTUAL AUDIO GENERATION, COMMUNICATION DEVICE, AND RELATED METHODS**
GEHÖRSCHUTZVORRICHTUNG MIT KONTEXTUELLER AUDIOERZEUGUNG, KOMMUNIKATIONSVORRICHTUNG UND ZUGEHÖRIGE VERFAHREN
APPAREIL DE PROTECTION AUDITIVE DOTÉ DE GÉNÉRATION AUDIO CONTEXTUELLE, DISPOSITIF DE COMMUNICATION ET PROCÉDÉS ASSOCIÉS

(30) Priority: 20.12.2019 EP 19218938
(43) Date of publication of application: 26.10.2022
(73) Proprietor: FalCom A/S, 2750 Ballerup (DK)
(72) Inventor: RAFT, Casper Silbo, 2750 Ballerup (DK); PEDERSEN, Søren Christian V., 2750 Ballerup (DK); CASTER, Jesse, 2750 Ballerup (DK)
(74) Representative: GN Store Nord A/S
(86) International application number: PCT/EP2020/087136
(87) International publication number: WO 2021/123246

(56) References cited:
- WO-A1-2018/138462
- US-A1- 2012 212 399
- US-A1- 2016 125 885
- US-A1- 2017 372 216
- US-A1- 2018 139 566
- US-A1- 2020 014 416

## Description

The present disclosure relates to a hearing protection apparatus and devices thereof including a communication device for a hearing protection apparatus and related methods including a method of operating a hearing protection apparatus.

### BACKGROUND

In combat situations or other stressed environments with a high noise level, it is desirable for a user to effectively protect his/her hearing while enabling the user to communicate with teammates via radio or other communication systems. During combat situations or other stressed environments, a user may need to wear a hearing protection device to attenuate noise, e.g. from gunfire, machinery and other types of constant or intermittent noise.

A drawback of using radio communication is that directional cues may be lost, causing the user to struggle in locating the source of an input signal, which may be dangerous in particular in combat situations, where the risk of friendly fire is heavily increased when directional cues are lost.

US 20180139566A1 relates to an augmented hearing system involving receiving, via an interface system, personnel location data indicating a location of at least one person and receiving, from an orientation system, headset orientation data corresponding with the orientation of a headset. First environmental element location data, indicating a location of at least a first environmental element, may be determined. Based at least in part on the headset orientation data, the personnel location data and the first environmental element location data, headset coordinate locations of at least one person and at least the first environmental element in a headset coordinate system corresponding with the orientation of the headset may be determined. An apparatus may be caused to provide spatialization indications of the headset coordinate locations. Providing the spatialization indications may involve controlling a speaker system to provide environmental element sonification corresponding with at least the first environmental element location data.

US 20160125885A1 relates to personal electronic devices (PEDs) having a sensory enhancement (SE) system for monitoring environmental conditions and detecting environmental events, for example but not limited to, changes in acoustic, thermal, optical, electromagnetic, chemical, dynamic, wireless, atmospheric, or biometric conditions. The detection of such events can be used to invoke a notification, an alert, a corrective action, or some other action, depending upon the implementation to the PED user or another party.

US 20120212399A1 et al. relates to an interactive head-mounted eyepiece with an integrated processor for handling content for display and an integrated image source for introducing the content to an optical assembly through which the user views a surrounding environment and the displayed content, wherein the optical assembly comprises an optically flat film, disposed at an angle in front of a user's eye, that reflects and transmits a portion of image light and transmits scene light from a see-through view of the surrounding environment, so that a combined image comprised of portions of the image light and the transmitted scene light is provided to a user's eye.

WO 2018/138462 A1 relates to A portable communication terminal for communicating on at least two independent radio networks, which comprises, in a single casing: - means (47, 49) for communication by time-multiplexing on a single radio channel, of the full-duplex TDMA type, for an audio communication in "conference" mode and "hands-free" on a first autonomous network without infrastructure, - means (43, 50) for accessing a half-duplex audio communication on a second network, that is autonomous and independent of the first full-duplex network, - a connection to a microphone (41) and a loudspeaker (52), - a wireless connection for a mobile telephone network, - at least one push-button (45) for switching the communication of the microphone and of the loudspeaker from and to the communication on the first network, the second network or the mobile telephone network, by means of electronic switching circuits, and - a Codec-type electronic mixer (42, 51) of audio signals, comprising a plurality of inputs and outputs, configured for mixing, on the local full-duplex network, half-duplex communications in order to provide to a user, via the microphone and the loudspeaker, simultaneous access to a plurality of audio communications on the various networks, as a function of pressing on the push-button.

### SUMMARY

Accordingly, there is a need for communication devices for hearing protection apparatuses and methods which increases the situational awareness of a user during combat situations or other stressed environments.

A communication device for a hearing protection apparatus is disclosed. The communication device comprises a processing unit, a memory, and an interface connected to the processing unit, the interface comprising a wired hearing protection device interface and a wired radio unit interface, wherein the communication device is configured to be body-worn. The processing unit is configured to obtain a first input via the interface, the first input comprising first audio input from a first microphone of a hearing protection device via the wired hearing protection interface and to obtain position data, via the interface and/or from a position module of the communication device. The processing unit is configured to determine a presence of an event based on the first input and the position data, wherein to determine a presence of an event comprises to determine first event data based on the first input. In accordance with a determination that an event is present, the processing unit is configured to determine a first audio output signal based on the first event data indicative of the event and the position data. The processing unit is configured to output the first audio output signal via the interface, wherein to output the first audio output signal via the interface comprises to output the first audio output signal to the hearing protection device.

Further, a method of operating a communication device for a hearing protection apparatus is provided, the communication device comprising a processing unit, memory, and an interface connected to the processing unit, wherein the interface comprises a wired hearing protection device interface and a wired radio unit interface, wherein the communication device is configured to be body-worn. The method comprises obtaining a first input via the interface, the first input comprising first audio input from a first microphone of a hearing protection device via the wired hearing protection interface and obtaining, via the interface and/or from a position module of the communication device, position data. The method comprises determining a presence of an event based on the first input and the position data, wherein determining a presence of an event comprises determining first event data based on the first input. The method comprises, in accordance with a determination that an event is present, determining a first audio output signal based on the first event data indicative of the event and the position data. The method comprises outputting the first audio output signal via the interface, wherein outputting the first audio output signal via the interface comprises outputting the first audio output signal to the hearing protection device.

Also disclosed is a server device for event monitoring in a communication system comprising the server device and a first communication device for a hearing protection apparatus. The first communication device may be a communication device as disclosed herein. The server device comprises one or more processing units, a memory, and an interface. The server device is configured to obtain first event data. The server device is configured to determine a presence of an event based on the first event data. The server device is configured to, in accordance with a determination that an event is present, determine a first input based on the first event data indicative of the event. The server device is configured to output the first input via the interface.

It is an advantage of the present disclosure that the situational awareness of a user of the communication device is enhanced in real-time, which in turn reduces decision time.

Thus, a user of the communication device, e.g. in a combat situation, experiences improved situational awareness and localization of events, e.g. audio source events such as gunshots, explosions, or vehicles, and allies/teammates, or other events types e.g. approaching a dangerous area such as an enemy territory or a minefield, and/or position of enemies, which is highly beneficial for the mission-performing user/warfighter operating in enemy territories/combat situations.

Further, the user, such as mission-performing user/warfighter, is provided with more precise and accurate localization/directional cues, so the mission-performing user/warfighter can accurately identify events and teammates in the surrounding area while staying protected with the hearing protection device and connected as well.

Further, directionality of input signal(s) from different sources can be improved, e.g. in turn providing a more effective spatial separation of sources in multi-source hearing protection devices.

### BRIEF DESCRIPTION OF THE DRAWINGS

The above and other features and advantages of the present invention will become readily apparent to those skilled in the art by the following detailed description of exemplary communication devices, methods, and/or server devices thereof with reference to the attached drawings, in which:
Fig. 1 schematically illustrates a mission-performing user wearing an exemplary hearing protection system comprising a communication device according to the disclosure,
Fig. 2 is a block diagram of an exemplary communication device according to the disclosure,
Fig. 3 is a flow diagram of an exemplary method according to the disclosure, and
Fig. 4 is a block diagram of an exemplary server device according to the disclosure.

### DETAILED DESCRIPTION

Various exemplary communication devices, methods, and/or server devices and details are described hereinafter, with reference to the figures when relevant. It should be noted that the figures may or may not be drawn to scale and that elements of similar structures or functions are represented by like reference numerals throughout the figures. It should also be noted that the figures are only intended to facilitate the description of the communication devices, methods, and/or server devices. They are not intended as an exhaustive description of the invention or as a limitation on the scope of the invention. In addition, an illustrated embodiment needs not have all the aspects or advantages shown. An aspect or an advantage described in conjunction with a particular embodiment is not necessarily limited to that embodiment and can be practiced in any other communication devices, methods, and/or server devices even if not so illustrated, or if not so explicitly described.

A communication device for a hearing protection apparatus is disclosed. The communication device comprises a processing unit, a memory, and an interface connected to the processing unit.

The processing unit is configured to obtain a first input via the interface. The first input may be one or more of a sound e.g. the sound from a vehicle, a weapon, or a teammate, a direction e.g. the direction from where a sound comes from and/or of where a position of interest is (e.g. an enemy territory), a distance to a sound or a position of interest, and/or a relative direction such as an angle indicating a range or radius of where a sound comes from or of where a position of interest is. The first input may be received from one or more microphones of a hearing protection apparatus. In other words, the first input may comprise first microphone input signal and/or second microphone input signal from respective first microphone and second microphone. The first input may be received from a server device via a radio unit connected to the interface. **In** other words, the first input may comprise a server device input.

The processing unit is configured to determine a presence of an event based on the first input. For example, the processing unit may determine the presence of the event indicative of an enemy gunshot from the front left of the user, based on the sound from the weapon shooting (e.g. as received by microphone(s) of the hearing protection apparatus), the position from where the sound was emitted (e.g. determined based on first input comprising audio input(s) from microphone(s)), and/or position data indicative of the position of the enemy (e.g. received from a server device via a radio unit connected to the interface). The position data may include a first position and/or a first orientation/direction of the hearing protection apparatus/device i.e. a first orientation/direction of the head of the user wearing the hearing protection device. The position data may thereby be indicative of a direction of the head of the user at the time where the first input and/or the second input are obtained. The position data may thereby be used as a reference direction with regards to a direction of an event. The first input and/or the second input may be linked to or correspond to a first orientation/direction, such that when the first input and the position data are obtained and/or transmitted (e.g. streamed to the server device), a server device may be able to obtain and/or determine the direction of the event with respect to the first orientation/direction of the head of the user. For example, a first direction of the head of the user may be linked to a first input and a second input, e.g. a set of a first audio input signal and a second audio input signal are obtained at times t1 and t2, and the first position data is received at a time t3, where t1 may be equal to t2, and t1<t3<t2 or t2<t3<t1. If the user does not change head direction, then one or more first input and/or second input may be linked to or correspond to the same first direction of the head of the user. The first input and/or the position data may be transmitted such that a server device is able will be able to distinguish or use first input, second input, and/or the position data from a plurality of users.

In accordance with a determination that an event is present, the processing unit is configured to determine a first audio output signal based on first event data indicative of the event. The first audio output signal may be an informative or contextual audio output giving an indication e.g. of the type of event of which the presence was determined. For example, the first audio output signal may be a sound alert indicating that an enemy fired a sniper shot. The first audio output signal may be a contextual audio output giving a contextual information indicative of the type of event that occurred and/or from which direction relative to the user the event occurred.

To determine a first audio output signal may comprise to generate a first audio output at the communication device and/or at the server device. To determine a first audio output signal may comprise to combine a first audio output signal generated at the communication device and a first audio output signal generated at the server device. The first audio output signal may be determined based on one or more or a combination of sound samples, sound pieces, or sound files stored on the memory of the communication device, sound samples, sound pieces, or sound files stored on the memory of the server device, and/or sound samples, sound pieces, or sound files generated based on the first input, at the communication device (e.g. the sound of a gunshot that is reproduced and outputted). The first audio output signal may comprise predetermined sound samples indicative of a given event and/or first event data based e.g. on a first audio output identifier, such as a given sound for a truck, a minefield, and/or an enemy. The first audio output signal may further comprise the combination of sound samples, such as combining a predetermined sound sample (such as a sound sample for indicating a truck), with a determined sound sample based on the position of the event (indicative of a direction of the event). For example, such a first audio output signal may comprise an alert saying, "vehicle from the right", which may be outputted e.g. through a right receiver of the hearing protection device of the user depending on the direction in which the user is looking i.e. the orientation of the hearing protection device. For example, if an event has a direction to the right of the user at the time where it occurred, and that the user turned his/her head to the right afterwards, the first audio output signal may be outputted such that the user receives the indication that the event is straight forward. The audio output signal may thereby "follow" the head direction of the use, if the head direction is changed.

The processing unit is configured to output the first audio output signal, e.g. to one or more receivers of a hearing protection device, via the interface.

The first input may comprise a direction of the event e.g. the direction from where the event occurred with respect to the direction of the head of the user, a distance to an event or a position of interest, and/or a relative direction such as an angle indicating a range or radius of where an event comes from or of where a position of interest is with respect to the hearing protection apparatus/device. The first input may for example be determined (such as estimated or calculated) with head-related transfer function, HRTF, derivations e.g. using a first audio input from a first microphone and a second audio input from a second microphone and/or first position data e.g. such as level difference, frequency difference, phase shifts, and/or time delays. By using HRTF derivations combined with first position data, an improved accuracy of localization of an event may be achieved, and front-back errors (errors in determining whether an event occurred in front of or behind the user) may be reduced or avoided. Accordingly, the processing unit may be configured to determine a direction of the event based on the first audio input and the second audio input.

To output the first audio output signal may comprise, prior to outputting the first audio output signal, to determine a first filter function based on a first direction and applying the first filter function to the first audio output signal. The first filter function may be a first head-related filter function such as a first left head-related filter function. By applying a filter function prior to outputting the first audio output and/or a second audio output may give a directional indication (e.g. simulating that the audio output comes from the direction of the event). In other words, the processor of the communication device may comprise a first filter module for filtering the first audio output signal according to the first filter function for provision of a first left output signal and a first right output signal.

In one or more exemplary communication devices, the processing unit is configured to determine event data, such as one or more or event type, event time, duration of event, direction of event and distance to event, based on the first input and/or the second input, and optionally include the event data in the first audio output signal.

The interface comprises a hearing protection device interface, such as one or more hearing protection device interfaces. The hearing protection device interfaces may allow the connection of the communication device to one or more hearing protection devices of the hearing protection apparatus of a mission-performing user, e.g. such that the first input or at least a part thereof is obtained from the one or more hearing protection devices.

The interface comprises a radio unit interface, such as one or more radio unit interfaces. The one or more radio unit interfaces may allow the connection of the communication device to one or more radio units of a hearing protection system of the user, e.g. such that the first input or at least a part thereof is obtained from the one or more radio units.

The interface comprises a hearing protection device interface and a radio unit interface, such as one or more hearing protection device interfaces and one or more radio unit interfaces. The hearing protection device interfaces may allow the connection of the communication device to one or more hearing protection devices of a hearing protection apparatus of the user, and the one or more radio unit interfaces may allow the connection of the communication device to one or more radio units of a hearing protection system of the user, e.g. such that the first input or at least a part thereof is obtained from the hearing protection device and the radio unit.

The hearing protection device interface and the radio unit interface are wired interfaces.

In one or more exemplary communication devices, methods, and/or server devices, the first input comprises first event data from a server device via the radio unit interface. The first event data may be determined and/or stored at the server device, such that to obtain the first input via the interface comprises to obtain first event data from the server device via the radio unit interface. The first event data may be indicative of the event of which the presence has been determined based on the first input.

The first input may be indicative of the event, where the event for example comprises an audio source event or a point of interest event. The audio source event may e.g. be a sound of interest for the user, such as a gunshot, an artillery fire, or a teammate mission-performing user communicating to the user. The point of interest event may for example be that the user is approaching or in movement towards a known area of interest such as a minefield, an enemy territory, a military base, or a predetermined arrival destination.

The first event data may be indicative of an audio source event or a point of interest event. The first event data may be data related to the event, such as parameters or information related to the event. The first event data may for example comprise an event position, a distance to an event position (e.g. an estimated distance from the position at which a gunshot has been fired or a distance to a minefield), an event type (e.g. a sound or a location), or an event direction (e.g. a direction to the event with respect to the user or an absolute direction with respect to a coordinate system, e.g. north, south, eat, west).

In one or more exemplary communication devices, methods, and/or server devices, the first input comprises first audio input from a first microphone via the hearing protection device interface. The first microphone may be a microphone of the hearing protection apparatus, for example of the hearing protection device. The first audio input may comprise first audio event data indicative of a first audio source event. The first audio input may comprise a first audio signal indicative of a first audio source event such as a gunshot sound, artillery fire sound, a driving vehicle sound, or a team mission-performing user speaking sound. The first audio input may be generated at the first microphone of the hearing protection apparatus, e.g. at a first microphone of the hearing protection device, based on the first audio source event. To determine the first audio output signal may comprise to determine the first audio output signal based on the first audio input.

In one or more exemplary communication devices, methods, and/or server devices, the processing unit is configured to obtain the first audio output signal from the memory, based on a first audio output identifier of the first event data.

In one or more exemplary communication devices, methods, and/or server devices, the processing unit is configured to output the first audio output signal via the interface.

The first audio output signal may e.g. be indicative of or corresponding to a given event (such as a gunshot, a vehicle, or a minefield) and/or a given first event data (e.g. an event direction, event position, event distance, or event type), or a combination thereof (e.g. a gunshot from the right/east, vehicle in a radius of 1 km, a minefield within 500m in a south-west direction), based on the first audio output identifier.

To output the first audio output signal via the interface may comprise to output a given first audio output signal (e.g. a given sound) from the memory, indicative of or corresponding to a given event and/or given first event data, based on the first audio output identifier. The first audio output signal may for example comprise a sound alert (for example if the user approaches a minefield or a gunshot position occurred in proximity of the user) or a guidance sound (for example to guide the user in direction of or away from a given event). The first audio output signal may vary in amplitude, pitch, etc, e.g. depending on the distance from the event or the type of event. For example, if the user is close to a gunshot, the first audio output signal may have a higher amplitude such that the user is made aware that the danger may be close, while still being hearing protected from the gunshot sound. The first audio output signal may inform the user of the type of event by having a first audio output identifier, e.g. by having pre-stored voice commands, alerts, or messages indicative of or corresponding to a given event and/or given first event data, that may be outputted to the user. The first audio output signal may give a directional cue or information of where the event and/or the first event data comes from e.g. with respect to the user, for example by outputting the first audio output signal via the interface to a first receiver of the hearing protection device and/or to a second receiver of the hearing protection device.

To output the first audio output signal via the interface comprises to output the first audio output signal to the hearing protection device, e.g. to a first receiver of the hearing protection device and/or to a second receiver of the hearing protection device.

The first receiver may be a left receiver and the second receiver may be a right receiver, such that the first audio output signal may be outputted to the right and/or the left receiver, e.g. at different amplitudes, level difference, frequency difference, phase shifts, and/or time delay depending on the direction of the event and/or the first event data.

In one or more exemplary communication devices, methods, and/or server devices, to determine a presence of an event, comprises to determine the first event data based on the first input. The first event data may either be received directly from the server or may be determined locally at the communication device.

To determine the first event data based on the first input may comprise to determine one or more of an event position, an event direction, and an event type, based on the first input. In other words, the first event data may comprise one or more of an event position, an event direction, and an event type.

To determine the first event data based on the first input, may comprise to determine the first event data based on the first input, at the communication device, without communicating with the server device.

To determine the first event data based on the first input may comprise to determine the first event data based on the first input, at the server device, where the first input is obtained from the communication device.

In one or more exemplary communication devices, methods, and/or server devices, the processing unit is configured to obtain a second input via the interface.

In one or more exemplary communication devices, methods, and/or server devices, the processing unit is configured to determine the presence of an event based on the second input.

In one or more exemplary communication devices, methods, and/or server devices, the second input comprises second event data from a server device via the radio unit interface.

The second event data may be determined and/or stored at the server device, such that to obtain the second input via the interface comprises to obtain second event data from the server device via the radio unit interface. The second event data may be indicative of the event of which the presence has been determined based on the second input.

The event may for example comprise an audio source event or a point of interest event.

The audio source event may e.g. be a sound of interest for the user, such as a gunshot, an artillery fire, or a teammate mission-performing user communicating to the user. The point of interest event may for example be that the user is approaching or in movement towards a known area of interest such as a minefield, an enemy territory, a military base, or a predetermined arrival destination.

The second event data may be indicative of an audio source event or a point of interest event. The second event data may be data related to the event, such as parameters or information related to the event. The second event data may for example comprise a distance to an event position (e.g. an estimated distance from the position at which a gunshot has been fired or a distance to a minefield), an event type (e.g. a sound or a location), or an event direction (e.g. a direction to the event with respect to the user or an absolute direction with respect to a coordinate system, e.g. north, south, eat, west).

In one or more exemplary communication devices, methods, and/or server devices, the second input comprises second audio input from a second microphone via the hearing protection device interface. The second microphone may be a microphone of the hearing protection apparatus, for example of the hearing protection device. The second audio input may comprise second audio event data indicative of a second audio source event. The second audio input may comprise a second audio signal indicative of a second audio source event such as a gunshot sound, artillery fire sound, a driving vehicle sound, or a team mission-performing user speaking sound. The second audio input may be generated at the second microphone of the hearing protection apparatus, e.g. at a second microphone of the hearing protection device, based on the second audio source event. To determine the second audio output signal may comprise to determine the second audio output signal based on the second audio input.

In one or more exemplary communication devices, methods, and/or server devices, to determine a presence of an event, comprises to determine the second event data based on the second input.

In one or more exemplary communication devices, methods, and/or server devices, the processing unit is configured to determine a second audio output signal based on second event data indicative of the event. In one or more exemplary communication devices, methods, and/or server devices, the processing unit is configured to output the first audio output signal and/or the second audio output signal via the interface.

The second audio output signal may e.g. be indicative of or corresponding to a given event (such as a gunshot, a vehicle, or a minefield) and/or a given second event data (e.g. an event direction, event position, or event type), or a combination thereof (e.g. a gunshot from the right/east, vehicle in a radius of 1 km, a minefield within 500m in a south-west direction), based on the second audio output identifier.

To output the second audio output signal may comprise, prior to outputting the second audio output signal, to determine a second filter function based on a second direction and applying the second filter function to the second audio output signal. The second filter function may be a second head-related filter function such as a first right head-related filter function. In other words, the processor of the communication device may comprise a filter module for filtering the second audio output signal according to the second filter function for provision of a second left output signal and a second right output signal.

To output the second audio output signal via the interface may comprise to output a given second audio output signal (e.g. a given sound) from the memory, indicative of or corresponding to a given event and/or given second event data, based on the second audio output identifier. The second audio output signal may for example comprise a sound alert (for example if the user approaches a minefield or a gunshot position occurred in proximity of the user) or a guidance sound (for example to guide the user in direction of or away from a given event). The second audio output signal may vary in amplitude, pitch, etc, e.g. depending on the distance from the event or the type of event. For example, if the user is close to a gunshot, the second audio output signal may have a higher amplitude such that the user is made aware that the danger may be close, while still being hearing protected from the gunshot sound. The second audio output signal may inform the user of the type of event, e.g. by having pre-stored voice commands, alerts, or messages indicative of or corresponding to a given event and/or given second event data, that may be outputted to the user. The second audio output signal may give a directional cue or information of where the event and/or the second event data comes from e.g. with respect to the user, for example by outputting the second audio output signal via the interface to a second receiver of the hearing protection device and/or to a second receiver of the hearing protection device.

To output the second audio output signal via the interface may comprise to output the second audio output signal to the hearing protection device, e.g. to a second receiver of the hearing protection device. It is to be noted that the first audio output signal and the second audio output signal are electrical output signals. The first audio output signal and the second audio output signal may be wireless signals.

The second receiver may be a right receiver and the first receiver may be a left receiver, such that the second audio output signal may be outputted to the right and/or the left receiver, e.g. at different amplitudes, depending on the direction of the event and/or the second event data.

In one or more exemplary communication devices, methods, and/or server devices the second input may be different from the first input. For example, the first input may comprise first audio input and the second input may comprise first event data.

In one or more exemplary communication devices, methods, and/or server devices the second input may be similar or identical to the first input.

For example, the first input may comprise first audio input and the second input may comprise second audio input, and/or the first input may comprise first primary audio input and the second input may comprise first secondary audio input.

In one or more exemplary communication devices, methods, and/or server devices, the second event data and/or second audio input may be based on an event and/or audio input that occurred after the event and/or audio input that the first event data and/or first audio input are based on.

The processing unit is configured to obtain position data via the interface and/or from a position module of the communication device.

In one or more exemplary communication devices, methods, and/or server devices, the processing unit is configured to obtain position data, via the interface and from the position module of the communication device.

An advantage of obtaining position data from the position module of the communication device may be that the position data of the user may be determined locally at the communication device. The position data may thereby be obtained in a faster manner and substantially instantaneously. By obtaining the position data from the position module of the communication device may allow to obtain the position of the user in a faster manner than obtaining the position data from a server device. The first direction of the event may be based on the position data and the determining of the presence of the event. By obtaining the position data from the position module of the communication device, the position data of the user may continuously (substantially without time delay) be obtained with respect to the position of the event and may thereby allow to determine the first direction of the event with respect to the user in a faster manner and depending on the movement of the user (e.g. the user looking in a direction or the user turning around with respect to the event).

A further advantage of obtaining position data from the position module of the communication device may be that the transferring of additional position data through the radio unit is avoided.

In one or more exemplary communication devices, methods, and/or server devices, to obtain position data comprises to receive a first position signal, via the interface and/or from the position module via the radio unit connected to the communication device.

In one or more exemplary communication devices, methods, and/or server devices, the position module and/or the radio unit may comprise a GPS sensor.

The processing unit is configured to determine the presence of an event based on the position data. The position data may be indicative of a first position of the position module and may comprise Global Positioning System (GPS) coordinates of the position module.

In one or more exemplary communication devices, methods, and/or server devices, to obtain position data comprises to receive user position data via the interface and/or from the position module of the communication device. The user position data may be indicative of a position of the user (e.g. as determined by the radio unit, the position module, and/or the server device) and may comprise Global Positioning System (GPS) coordinates of the radio unit, the position module, and/or communication device. Accordingly, the communication device may comprise a position module for determining a position, such as GPS position, of the communication device.

In one or more exemplary communication devices, methods, and/or server devices, the position data comprises first direction data indicative of a first direction of the event. To determine a first audio output signal may comprise to determine the first audio output signal based on the position data. To determine a first audio output signal may comprise to determine the first audio output signal based on first direction data indicative of a first direction of the event.

In one or more exemplary communication devices, methods, and/or server devices, the position data may comprise second direction data indicative of a head direction of a user of the communication device. To determine a first audio output signal may comprise to determine the first audio output signal based on second direction data indicative of a head direction of a user of the communication device.

In one or more exemplary communication devices, methods, and/or server devices, the position data may comprise data from a position module on the hearing protection device, e.g. on the head of the user. To determine a first audio output signal may comprise to determine the first audio output signal based on data from a position module on the hearing protection device, e.g. on the head of the user.

In one or more exemplary communication devices, methods, and/or server devices, to determine the presence of an event comprises to determine an angle between a head direction of the user and a first direction of the event.

The processing unit is configured to obtain, e.g. via the interface and/or a position module of the communication device, a user position signal indicative of a position of the user.

The processing unit is configured to obtain, e.g. via the interface and/or the position module, a direction signal indicative of a head direction of the user. The position module may comprise a first accelerometer and a second accelerometer.

Optionally, the position module may comprise a plurality of position modules, such as a first position module to provide a location of the user, and a second position module to provide a direction tracking (e.g. a head tracker) indicative of the direction in which the user looks or is oriented.

The processing unit is configured to determine a first angle, between the head direction and a first direction, from the user to the event.

The communication device may operate irrespectively from a server device. **In** other words, the communication device may operate autonomously from a server device. For example, a plurality of communication devices e.g. of a plurality of users (a group of mission performing users such as a military unit, a police unit, a special force unit, and/or a firefighter unit) may be configured to operate irrespectively of a server device. **In** other words, the plurality of communication devices may be configured to operate and communicate with one another without communicating via a server device.

A method of operating a hearing protection apparatus comprising a processing unit, memory, and an interface connected to the processing unit is disclosed. The method comprises obtaining a first input via the interface. The method comprises determining a presence of an event based on the first input. The method comprises, in accordance with a determination that an event is present, determining a first audio output signal based on first event data indicative of the event. The method comprises outputting the first audio output signal via the interface.

A server device for event monitoring in a communication system comprising the server device and a first communication device for a hearing protection apparatus is disclosed. The server device comprises one or more processing units, a memory, and an interface. The server device is configured to obtain first event data. The server device is configured to determine a presence of an event based on the first event data. The server device is configured to, in accordance with a determination that an event is present, determine a first input based on the first event data indicative of the event. The server device is configured to output the first input via the interface.

To obtain first event data may comprise to obtain first event data from one or more communication devices. For example, in a multi-user system where a plurality of user-performing users having a plurality of communication devices communicating with the server device, the server device may be configured to obtain first event data from the plurality of communication devices. To determine a first input may therefore comprise to determine a plurality of first inputs for the plurality of users. The server device may be configured to output the first inputs via the interface.

The server device is optionally configured to determine a presence of a first event based on first event data, and to determine a presence of a second event based on second event data. To output the first input may comprise transmitting first input indicative of the first event to the first communication device and/or to the second communication device. To output first input may comprise transmitting second input indicative of the second event to the first communication device and/or to the second communication device.

It is to be understood that a description of a feature in relation to communication device(s) is also applicable to the corresponding method(s) and server device(s) and vice versa.

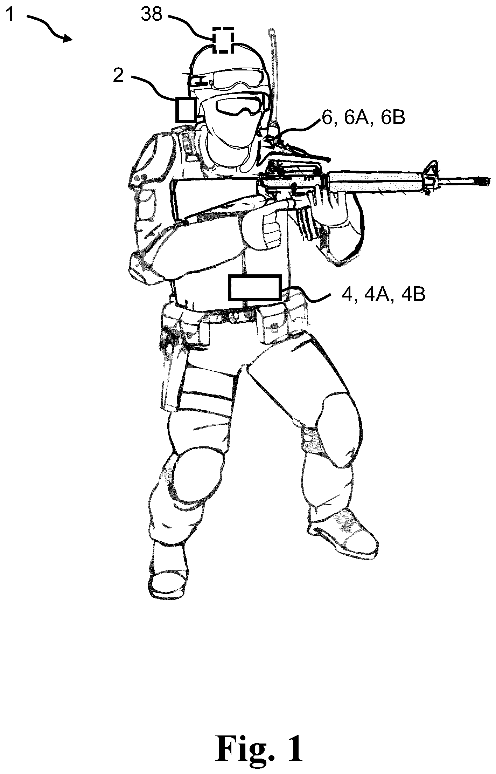

Fig. 1 shows an exemplary mission-performing user 1 wearing an exemplary hearing protection system comprising a communication device 4 according to the disclosure, a hearing protection device 2, and a radio unit 6. In one or more exemplary communication devices, the hearing protection system may comprise a position module 38, that the user may wear e.g. as shown in the figure on his helmet. The communication device 4 is here positioned on the torso of the user. The communication device may alternatively be worn on other body-parts of the user, such as the back, the arm, the leg, and/or the head (e.g. the helmet).

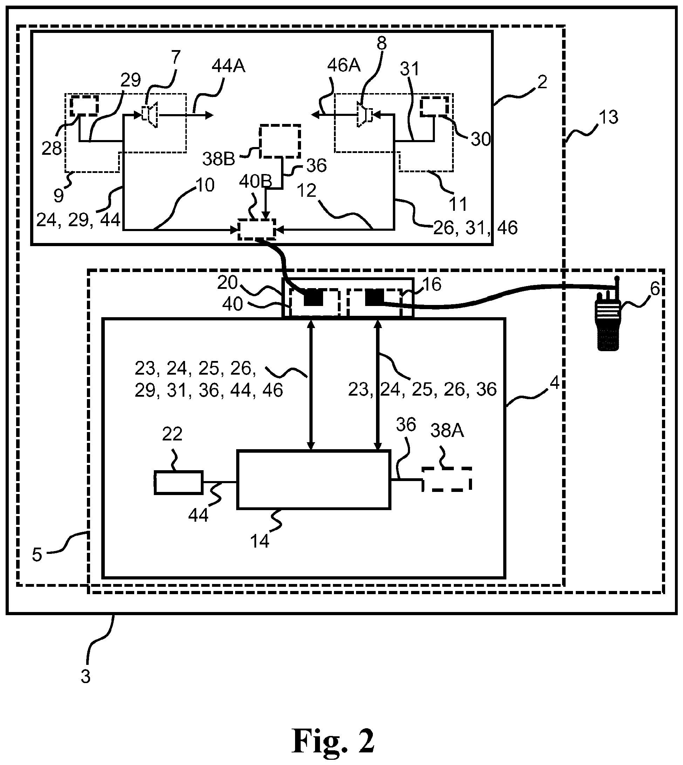

Fig. 2 shows an exemplary hearing protection system 3 comprising a communication device 4 according to the disclosure, a hearing protection device 2, and a radio unit 6. The communication device 4 comprises a processing unit 14, a memory 22, and an interface 20 connected to the processing unit 14. The processing unit 14 is configured to obtain a first input 23 via the interface 20. In one or more exemplary communication devices, the communication device 4 and the radio unit may be comprised in a communication system 5. In one or more exemplary communication devices, the hearing protection device 2 comprises a hearing protection device interface 40.

The processing unit 14 is configured to determine a presence of an event based on the first input 23. In accordance with a determination that an event is present, the processing unit 14 is configured to determine a first audio output signal 44 based on first event data indicative of the event. The processing unit 14 is configured to output the first audio output signal 44 via the interface 20.

In one or more exemplary communication devices, the interface 20 comprises a hearing protection device interface 40. The hearing protection device interface 40 may allow the connection of the communication device 4 to the hearing protection device 2 of the hearing protection apparatus 13 of a mission-performing user 1, e.g. such that the first input 23 is obtained from the hearing protection device 2, through (or partly through) a first input signal 24.

In one or more exemplary communication devices, the interface 20 comprises a radio unit interface 16. The radio unit interface 16 may allow the connection of the communication device 4 to the radio unit 6 of a hearing protection system 3 or a communication system 5 of the user, e.g. such that the first input 23 is obtained from the radio unit 6, through (or partly through) a first input signal 24.

In one or more exemplary communication devices, the interface 20 comprises a hearing protection device interface 40 and a radio unit interface 16. The hearing protection device interface 40 may allow the connection of the communication device 4 to the hearing protection device 2 of the hearing protection apparatus 13 of the user 1, and the radio unit interface 16 may allow the connection of the communication device 4 to the radio unit 6 of the hearing protection system 3 of the user, e.g. such that the first input 23 is obtained from the hearing protection device 2 and the radio unit 6, through (or partly through) a first input signal 24 through the hearing protection device interface 40 and the radio unit interface 16.

In one or more exemplary communication devices, the first input 23 comprises first event data from a server device via the radio unit interface 16.

The first event data may be determined and/or stored at the server device, such that to obtain the first input 23 via the interface 20 comprises to obtain first event data from the server device via the radio unit interface 16 from the radio unit 6.

In one or more exemplary communication devices, the first input 23 comprises a first audio input 29 from a first microphone 28 via the hearing protection device interface 40. The first microphone may be a microphone of the hearing protection apparatus 13, for example of the hearing protection device 2. The first audio input 29 may be generated at the first microphone 28 of the hearing protection apparatus 13, e.g. at the first microphone 28 of the hearing protection device 2, based on the first audio source event. To determine the first audio output signal 44 may comprise to determine the first audio output signal 44 based on the first audio input 28.

In one or more exemplary communication devices, the processing unit 14 is configured to obtain the first audio output signal 44 from the memory 22, based on a first audio output identifier of the first event data.

In one or more exemplary communication devices, the processing unit 14 is configured to output the first audio output signal 44 via the interface 20.

To output the first audio output signal 44 via the interface 20 may comprise to output the first audio output signal 44 to the hearing protection device 2, e.g. to a first receiver 7 of the hearing protection device 2. The first receiver 7 may be comprised in a first ear protector 9, such as a first earpiece or a first earmuff, of the hearing protection device 2, the first earpiece 9 comprising a first connection 10, such as a left connection.

The first receiver 7 may be a left receiver, such that the first audio output signal 44 may be outputted to the left receiver, e.g. at different amplitudes, depending on the direction of the event and/or the first event data. The first audio output signal 44 may be outputted by the first receiver 7 as a first audio output 44A.

In one or more exemplary communication devices, the processing unit 14 is configured to obtain a second input 25 via the interface 20. In one or more exemplary communication devices, the processing unit 14 is configured to determine the presence of an event based on the second input 25.

In one or more exemplary communication devices, the second input 25 comprises second event data from a server device via the radio unit interface 16.

In one or more exemplary communication devices, the second input 25 comprises second audio input 31 from a second microphone 30 via the hearing protection device interface 40. The second microphone 30 may be a microphone of the hearing protection apparatus 13, for example of the hearing protection device 2. The second input signal 31 may comprise second audio event data indicative of a second audio source event.

In one or more exemplary communication devices, to determine a presence of an event, comprises to determine the second event data based on the second input 25.

In one or more exemplary communication devices, the processing unit 14 is configured to determine a second audio output signal 46 based on second event data indicative of the event.

In one or more exemplary communication devices, the processing unit 14 is configured to output the first audio output signal 44 and/or the second audio output signal 46 via the interface 20.

The second audio input 31 may be generated at the second microphone 30 of the hearing protection apparatus 13, e.g. at the second microphone 30 of the hearing protection device 2, based on the second audio source event. To determine the second audio output signal 46 may comprise to determine the second audio output signal 46 based on the second audio input 31.

To output the second audio output signal 46 via the interface 20 may comprise to output the second audio output signal 46 to the hearing protection device 2, e.g. to a second receiver 8 of the hearing protection device 2. The second receiver 8 may be comprised in a second ear protector 11, such as a second earpiece or second earmuff, of the hearing protection device 2, the second earpiece 11 comprising a second connection 12, such as a right connection. The connection 10 and 12 might be wired connections or wireless connections such as Bluetooth connections.

The second receiver 8 may be a right receiver, such that the second audio output signal 46 may be outputted to the right receiver, e.g. at different amplitudes, depending on the direction of the event and/or the first event data. The second audio output signal 46 may be outputted by the second receiver 8 as a second audio output 46A.

In one or more exemplary communication devices, the processing unit 14 is configured to obtain position data 36 from a communication device position module 38A.

In one or more exemplary communication devices, the processing unit 14 is configured to obtain position data 36, via the hearing protection device interface 40 from a hearing protection device position module 38B.

In one or more exemplary communication devices, the processing unit 14 is configured to obtain position data 36, via the radio unit interface 16 via the radio unit 6 from the server device.

In one or more exemplary communication devices, the processing unit 14 is configured to determine the presence of an event based on the position data 36.

In one or more exemplary communication devices, the position data 36 may comprise data from a position module 38, 38B, e.g. on the head of the user 1. The position module may alternatively be positioned on other body parts of the user, such as arranged with the radio unit or other parts of the communication system (e.g. outside/separately from the communication device and/or the hearing protection device).

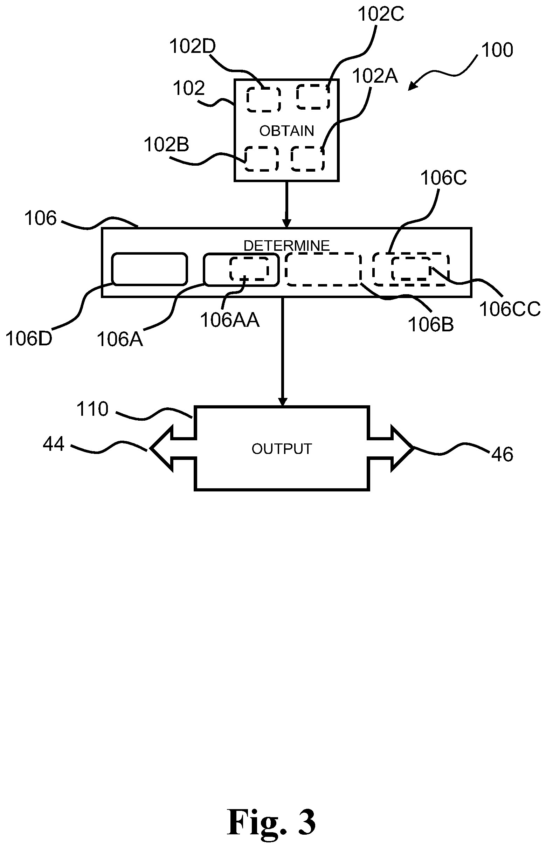

Fig. 3 is a flow diagram of an exemplary method of operating a hearing protection apparatus comprising a processing unit, a memory, and an interface.

The method 100 comprises obtaining 102 a first input via the interface.

In one or more exemplary methods, obtaining 102 the first input comprises obtaining 102A first event data from the server device via the radio unit interface from the radio unit.

In one or more exemplary methods, the method 100 comprises obtaining 102B a first audio output signal from the memory, based on a first audio output identifier of the first event data.

In one or more exemplary methods, the method 100 comprises obtaining 102C a second input via the interface.

In one or more exemplary methods, the method 100 comprises obtaining 102D position data via the interface.

The method 100 comprises determining 106 a presence of an event based on the first input.

In one or more exemplary methods, determining 106 a presence of an event comprises determining 106B the first event data based on the first input.

In one or more exemplary methods, the method 100 comprises determining 106C a presence of an event based on the second input.

In one or more exemplary methods, determining 106 a presence of an event comprises determining 106CC the second event data based on the second input.

The method 100 comprises, in accordance with a determination 106 that an event is present, determining 106A a first audio output signal based on first event data indicative of the event.

In one or more exemplary methods, determining 106A the first audio output signal comprises determining 106AA the first audio output signal based on the first audio input.

In one or more exemplary methods, the method comprises determining 106D the presence of an event based on the position data.

The method comprises outputting 110 the first audio output signal via the interface.

In one or more exemplary methods, the method comprises outputting 110 the second audio output signal 46 via the interface.

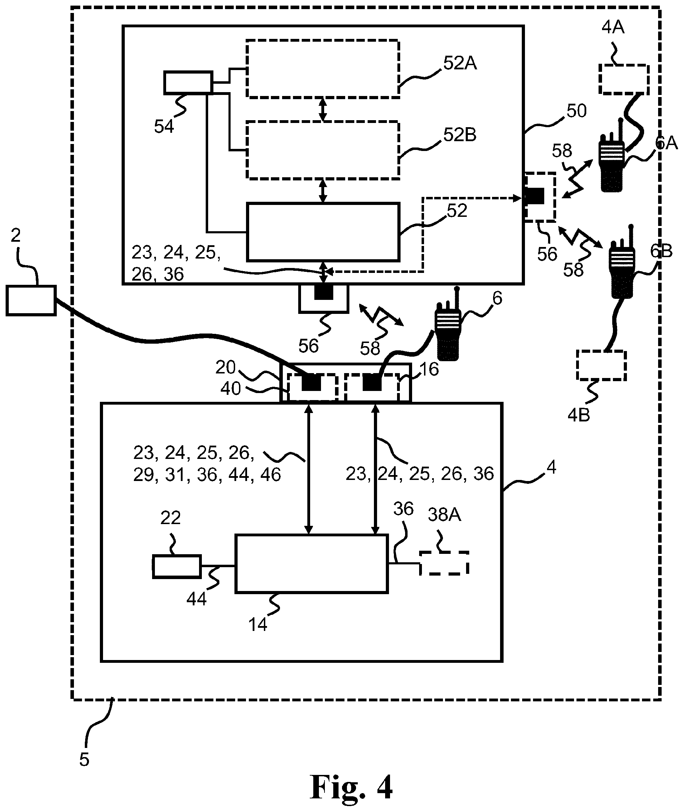

Fig. 4 shows an exemplary server device 50 for event monitoring in a communication system 5 comprising the server device 50 and a first communication device 4 for a hearing protection apparatus. The server device 50 comprises one or more processing units 52, 52A, 52B, a memory 54, and an interface 56. The server device 50 is configured to obtain first event data. The server device 50 is configured to determine a presence of an event based on the first event data. The server device 50 is configured to, in accordance with a determination that an event is present, determine a first input 23 based on the first event data indicative of the event. The server device 50 is configured to output the first input 23 via the interface 56.

In one or more exemplary server devices, the server device 50 is configured to output the first input 23 via the interface 56 to a radio unit 6, through a connection 58. The connection 58 may be wireless, such as a radio connection, or a wired connection.

In one or more exemplary server devices, the server device 50 is configured to output the first input 23 via the interface 56 through a first input signal 24.

In one or more exemplary server devices, the server device 50 is configured to obtain second event data. The server device 50 is configured to determine a presence of an event based on the second event data. The server device 50 is configured to, in accordance with a determination that an event is present, determine a second input 25 based on the second event data indicative of the event. The server device 50 is configured to output the second input 25 via the interface 56. The server device 50 is configured to output the second input 25 via the interface 56 to a radio unit 6, through a connection 58. The server device 50 is configured to output the second input 25 via the interface 56 through a second input signal 26.

In one or more exemplary server devices, the server device 50 is configured to obtain position data 56 via the interface 56. The server device 50 is configured to determine a presence of an event based on the position data 56.

In one or more exemplary server devices, the server device 50 is configured to output the first input 23 via the interface 56 to a second radio unit 6A configured to communicate with a second communication device 4A, and a third radio unit 6B configured to communicate with a third communication device 4B.

In one or more exemplary server devices, the server device 50 is configured to output the first input 23 via the interface 56 through a first input signal 24.

For example, in a multi-user system where a plurality of mission-performing users each having a communication device communicating with the server device, the server device may be configured to obtain first event data from one or more of the plurality of communication devices. To determine a first input may therefore comprise to determine a first input for each or some of the plurality of users. The server device may be configured to output the first inputs via the interface.

The use of the terms "first", "second", "third" and "fourth", "primary", "secondary", "tertiary" etc. does not imply any particular order, but are included to identify individual elements. Moreover, the use of the terms "first", "second", "third" and "fourth", "primary", "secondary", "tertiary" etc. does not denote any order or importance, but rather the terms "first", "second", "third" and "fourth", "primary", "secondary", "tertiary" etc. are used to distinguish one element from another. Note that the words "first", "second", "third" and "fourth", "primary", "secondary", "tertiary" etc. are used here and elsewhere for labelling purposes only and are not intended to denote any specific spatial or temporal ordering.

Furthermore, the labelling of a first element does not imply the presence of a second element and vice versa. It may be appreciated that Figs. 1-4 comprise some modules or operations which are illustrated with a solid line and some modules or operations which are illustrated with a dashed line. The modules or operations which are comprised in a solid line are modules or operations which are comprised in the broadest example embodiment. The modules or operations which are comprised in a dashed line are example communication devices, methods, and/or server devices which may be comprised in, or a part of, or are further modules or operations which may be taken in addition to the modules or operations of the solid line example communication devices, methods, and/or server devices. It should be appreciated that these operations need not be performed in order presented. Furthermore, it should be appreciated that not all of the operations need to be performed.

It is to be noted that the word "comprising" does not necessarily exclude the presence of other elements or steps than those listed.

It is to be noted that the words "a" or "an" preceding an element do not exclude the presence of a plurality of such elements.

It should further be noted that any reference signs do not limit the scope of the claims, that the exemplary communication devices, methods, and/or server devices may be implemented at least in part by means of both hardware and software, and that several "means", "units" or "devices" may be represented by the same item of hardware.

The various exemplary methods, devices, and systems described herein are described in the general context of method steps processes, which may be implemented in one aspect by a computer program product, embodied in a computer-readable medium, including computer-executable instructions, such as program code, executed by computers in networked environments. A computer-readable medium may include removable and non-removable storage devices including, but not limited to, Read Only Memory (ROM), Random Access Memory (RAM), compact discs (CDs), digital versatile discs (DVD), etc. Generally, program modules may include routines, programs, objects, components, data structures, etc. that perform specified tasks or implement specific abstract data types. Computer-executable instructions, associated data structures, and program modules represent examples of program code for executing steps of the methods disclosed herein. The particular sequence of such executable instructions or associated data structures represents examples of corresponding acts for implementing the functions described in such steps or processes.

Although features have been shown and described, it will be understood that they are not intended to limit the claimed invention. The specification and drawings are, accordingly, to be regarded in an illustrative rather than restrictive sense.

### LIST OF REFERENCES

1 mission-performing user
2 hearing protection device
3 hearing protection system
4, 4A, 4B communication device
5 communication system
6, 6A, 6B radio unit
7 first receiver
8 second receiver
9 first ear protector, first earpiece, first earmuff
10 first connection
11 second ear protector, second earpiece, second earmuff
12 second connection
13 hearing protection apparatus
14 processing unit
16 radio unit interface
20 interface
22 memory
23 first input
24 first input signal
25 second input
26 second input signal
28 first microphone
29 first audio input
30 second microphone
31 second audio input
36 position data
38 position module
38A communication device position module
38B hearing protection device position module
40, 40B hearing protection device interface
44 first audio output signal
44A first audio output
46 second audio output signal
46A second audio output
50 server device
52, 52A, 52B processing units
54 memory
56 interface
58 connection
100 method of operating a hearing protection apparatus
102 obtaining a first input via the interface
102A obtaining first event data from the server device via the radio unit interface from the radio unit
102B obtaining a first audio output signal from the memory, based on a first audio output identifier of the first event data
102C obtaining a second input via the interface
102D obtaining position data via the interface
106 determining a presence of an event based on the first input
106A in accordance with a determination that an event is present, determining a first audio output signal based on first event data indicative of the event
106AA determining the first audio output signal based on the first audio input
106B determining the first event data based on the first input
106C determining a presence of an event based on the second input
106CC determining the second event data based on the second input
106D determining the presence of an event based on the position data
110 outputting the first audio output signal via the interface

## Claims

1. A communication device (4, 4A, 4B) for a hearing protection apparatus, the communication device (4, 4A, 4B) comprising
- a processing unit (14);
- a memory (22); and
- an interface (20) connected to the processing unit (14), the interface (20) comprising a wired hearing protection device interface (40, 40B) and a wired radio unit interface (16),
wherein the communication device (4, 4A, 4B) is configured to be body-worn, and wherein the processing unit (14) is configured to:
- obtain a first input (23) via the interface (20), the first input comprising first audio input (29) from a first microphone of a hearing protection device via the wired hearing protection interface;
- obtain position data (36), via the interface (20) and/or from a position module (38) of the communication device (4, 4A, 4B);
- determine a presence of an event based on the first input (23) and the position data (36), wherein to determine the presence of the event comprises to determine first event data based on the first input (23);
- in accordance with a determination that the event is present, determine a first audio output signal (44) based on the first event data indicative of the event and the position data (36); and
- output the first audio output signal (44) via the interface (20), wherein to output the first audio output signal via the interface (20) comprises to output the first audio output signal to the hearing protection device.

2. Communication device (4, 4A, 4B) according to claim 1, wherein the processing unit (14) is configured to:
- obtain a second input (25) via the interface (20); and
- determine the presence of the event based on the second input (25).

3. Communication device (4, 4A, 4B) according to claim 2, wherein the second input (25) comprises second event data from a server device via the wired radio unit interface (16).

4. Communication device (4, 4A, 4B) according to any one of claims 2-3, wherein the second input (25) comprises second audio input (31) from a second microphone (30) via the wired hearing protection device interface (40, 40B).

5. Communication device (4, 4A, 4B) according to claim 3 or claim 4 as dependent on claim 3, wherein the processing unit (14) is configured to:
- determine a second audio output signal (46) based on the second event data indicative of the event; and
- output the second audio output signal (46) via the interface (20).

6. Communication device (4, 4A, 4B) according to any one of claims 1-5, wherein the position data (36) comprises first direction data indicative of a first direction of the event.

7. Communication device (4, 4A, 4B) according to any one of claims 1-6, wherein the processing unit (20) is configured to obtain the first audio output signal (44) from the memory (22) based on a first audio output identifier of the first event data.

8. A method (100) for operating a communication device of a hearing protection apparatus, the communication device comprising a processing unit, a memory, and an interface connected to the processing unit, wherein the interface comprises a wired hearing protection device interface and a wired radio unit interface, wherein the communication device is configured to be body-worn; the method (100) comprising:
- obtaining (102) a first input via the interface, the first input comprising first audio input from a first microphone of a hearing protection device via the wired hearing protection interface;
- obtaining, via the interface and/or from a position module of the communication device, position data;
- determining (106) a presence of an event based on the first input and the position data, wherein determining (106) a presence of an event comprises determining first event data based on the first input;
- in accordance with a determination that an event is present, determining (106A) a first audio output signal based on the first event data indicative of the event and the position data; and
- outputting (110) the first audio output signal via the interface, wherein outputting (110) the first audio output signal via the interface comprises outputting the first audio output signal to the hearing protection device.

## Patentansprüche

1. Ein Kommunikationsgerät (4, 4A, 4B) für ein Gehörschutzgerät, das Kommunikationsgerät (4, 4A, 4B) besteht aus
- eine Verarbeitungseinheit (14);
- eine Erinnerung (22); und
- eine Schnittstelle (20), die an die Verarbeitungseinheit angeschlossen ist (14), die Schnittstelle (20) besteht aus einer kabelgebundenen Gehörschutzschnittstelle, die (40, 40B) und einer kabelgebundenen Funkeinheitsschnittstelle (16),
wobei das Kommunikationsgerät (4, 4A, 4B) so konfiguriert ist, dass es am Körper getragen wird, und
wobei die Verarbeitungseinheit (14) konfiguriert ist zu:
- einen ersten Eingang (23) über die Schnittstelle (20) zu erhalten, wobei der erste Eingang aus dem ersten Audioeingang (29) vom ersten Mikrofon eines Hörschutzgeräts über die kabelgebundene Hörschutzschnittstelle besteht;
- Positionsdaten (36) über die Schnittstelle (20) und/oder von einem Positionsmodul (38) des Kommunikationsgeräts (4, 4A, 4B) zu erhalten;
- das Vorhandensein eines Ereignisses anhand der ersten Eingabe (23) und der Positionsdaten (36) bestimmen, wobei die Bestimmung des Ereignisses darin besteht, die Daten des ersten Ereignisses auf Basis der ersten Eingabe zu bestimmen (23);
- gemäß der Feststellung, dass das Ereignis vorhanden ist, ein erstes Audiosignal (44) auf Basis der Erstereignisdaten bestimmen, die das Ereignis und die Positionsdaten anzeigen (36); und
- das erste Audio-Ausgangssignal (44) über die Schnittstelle (20) auszugeben, wobei die Ausgab des ersten Audio-Ausgangssignals über die Schnittstelle (20) darin besteht, das erste Audio-Ausgangssignal an das Gehörschutzgerät auszugeben.

2. Kommunikationsgerät (4, 4A, 4B) gemäß Anspruch 1, wobei die Verarbeitungseinheit (14) konfiguriert ist zu:
- einen zweiten Eingang (25) über die Schnittstelle erhalten (20); und
- bestimmen Sie das Vorhandensein des Ereignisses anhand der zweiten Eingabe (25).

3. Kommunikationsgerät (4, 4A, 4B) gemäß Anspruch 2, wobei der zweite Eingang (25) aus Zweit-Ereignisdaten eines Servergeräts über die kabelgebundene Funkschnittstelle besteht (16).

4. Kommunikationsgerät (4, 4A, 4B) gemäß einem der Ansprüche 2-3, wobei der zweite Eingang (25) den zweiten Audioeingang (31) eines zweiten Mikrofons (30) über die kabelgebundene Hörschutzschnittstelle-Schnittstelle (40, 40B) umfasst.

5. Kommunikationsgerät (4, 4A, 4B) gemäß Anspruch 3 oder Anspruch 4 als abhängig von Anspruch 3, wobei die Verarbeitungseinheit (14) konfiguriert ist zu:
- ein zweites Audio-Ausgangssignal (46) basierend auf den zweiten Ereignisdaten bestimmen, die das Ereignis anzeigen; und
- das zweite Audiosignal (46) über das Interface (20) auszugeben.

6. Kommunikationsgerät (4, 4A, 4B) gemäß einem der Ansprüche 1-5, wobei die Positionsdaten (36) aus Erstrichtungsdaten bestehen, die auf eine erste Richtung des Ereignisses hinweisen.

7. Kommunikationsgerät (4, 4A, 4B) gemäß einem der Ansprüche 1-6, wobei die Verarbeitungseinheit (20) so konfiguriert wird, dass sie das erste Audio-Ausgangssignal (44) aus dem Speicher (22) basierend auf einer ersten Audioausgabe-Kennung der ersten Ereignisdaten abruft.

8. Eine Methode (100) zum Betrieb eines Kommunikationsgeräts eines Gehörschutzgeräts, eines Kommunikationsgeräts aus einer Verarbeitungseinheit, einem Speicher und einer Schnittstelle, die mit der Verarbeitungseinheit verbunden ist, wobei die Schnittstelle eine kabelgebundene Schnittstelle für Gehörschutzgeräte und eine kabelgebundene Funkeinheit umfasst, bei der das Kommunikationsgerät so konfiguriert ist, dass es am Körper getragen werden kann; die Methode (100) umfasst:
- Erhalt (102) eines ersten Eingangs über das Interface, wobei der erste Eingang den ersten Audioeingang eines ersten Mikrofons eines Gehörschutzgeräts über die kabelgebundene Hörschutzschnittstelle umfasst;
- Erfassung von Positionsdaten über die Schnittstelle und/oder von einem Positionsmodul des Kommunikationsgeräts;
- Bestimmung (106) der Präsenz eines Ereignisses auf Basis der ersten Eingabe und der Positionsdaten, wobei die Bestimmung (106) die Anwesenheit eines Ereignisses die Bestimmung der Daten des ersten Ereignisses auf Basis der ersten Eingabe umfasst;
- gemäß der Feststellung, dass ein Ereignis vorhanden ist, wobei (106A) ein erstes Audiosignal auf Basis der ersten Ereignisdaten bestimmt wird, die das Ereignis und die Positionsdaten anzeigen; und
- Ausgabe (110) des ersten Audio-Ausgangssignals über das Interface, wobei (110) das erste Audio-Ausgangssignal über die Schnittstelle das erste Audio-Ausgangssignal an das Hörschutzgerät ausgibt.

## Revendications

1. Un dispositif de communication (4, 4A, 4B) pour un appareil de protection auditive, le dispositif de communication (4, 4A, 4B) comprenant
- une unité de traitement (14) ;
- un souvenir (22) ; et
- une interface (20) connectée à l'unité de traitement (14), l'interface (20) comprenant une interface de dispositif de protection auditive filaire (40, 40B) et une interface d'unité radio filaire (16),
où le dispositif de communication (4, 4A, 4B) est configuré pour être porté sur le corps, et
où l'unité de traitement (14) est configurée pour :
- obtenir une première entrée (23) via l'interface (20), la première entrée comprenant la première entrée audio (29) provenant du premier microphone d'un dispositif de protection auditive via l'interface de protection auditive filaire ;
- obtenir des données de position (36), via l'interface (20) et/ou depuis un module de position (38) du dispositif de communication (4, 4A, 4B) ;
- déterminer la présence d'un événement à partir de la première entrée (23) et des données de position (36), où déterminer la présence de l'événement comprend de déterminer les données du premier événement à partir de la première entrée (23) ;
- conformément à la détermination de la présence de l'événement, déterminer un premier signal audio de sortie (44) basé sur les données du premier événement indiquant l'événement et les données de position (36) ; et
- Sortir le premier signal de sortie audio (44) via l'interface (20), où pour sortir le premier signal audio via l'interface (20), il faut envoyer le premier signal de sortie audio vers le dispositif de protection auditive.

2. Dispositif de communication (4, 4A, 4B) selon la revendication 1, où l'unité de traitement (14) est configurée pour :
- obtenir une seconde entrée (25) via l'interface (20) ; et
- déterminer la présence de l'événement à partir de la seconde entrée (25).

3. Dispositif de communication (4, 4A, 4B) selon la revendication 2, où la seconde entrée (25) comprend les données du second événement provenant d'un dispositif serveur via l'interface radio filaire (16).

4. Dispositif de communication (4, 4A, 4B) selon l'une des revendications 2 à 3, où la seconde entrée (25) est constituée d'une seconde entrée audio (31) provenant d'un second microphone (30) via l'interface du dispositif de protection auditive filaire (40, 40B).

5. Dispositif de communication (4, 4A, 4B) selon la revendication 3 ou la revendication 4 comme dépendant de la revendication 3, où l'unité de traitement (14) est configurée pour :
- déterminer un second signal de sortie audio (46) basé sur les données du second événement indiquant l'événement ; et
- Sortir le second signal de sortie audio (46) via l'interface (20).

6. Dispositif de communication (4, 4A, 4B) selon l'une des revendications 1 à 5, où les données de position (36) comprennent des données de première direction indiquant une première direction de l'événement.

7. Dispositif de communication (4, 4A, 4B) selon l'une des revendications 1 à 6, où l'unité de traitement (20) est configurée pour obtenir le premier signal de sortie audio (44) de la mémoire (22) à partir d'un identifiant de sortie audio de la première donnée d'événement.

8. Une méthode (100) pour faire fonctionner un dispositif de communication d'un appareil de protection auditive, le dispositif de communication comprenant une unité de traitement, une mémoire et une interface connectée à l'unité de traitement, dans laquelle l'interface comprend une interface de dispositif de protection auditive filaire et une interface radio filaire, où le dispositif de communication est configuré pour être porté sur le corps ; la méthode (100) comprend :
- obtenir (102) une première entrée via l'interface, la première entrée comprenant la première entrée audio provenant d'un premier microphone d'un dispositif de protection auditive via l'interface filaire de protection auditive ;
- obtenir, via l'interface et/ou à partir d'un module de position du dispositif de communication, des données de position ;
- déterminer (106) la présence d'un événement à partir de la première entrée et des données de position, où déterminer (106) la présence d'un événement comprend déterminer les données du premier événement à partir de la première entrée ;
- conformément à la détermination qu'un événement est présent, déterminant (106A) un premier signal audio de sortie basé sur les premières données d'événement indiquant l'événement et les données de position ; et
- l'envoi (110) du premier signal audio via l'interface, où l'envoi (110) du premier signal audio via l'interface consiste à envoyer le premier signal audio vers le dispositif de protection auditive.
